Europäisches Patentamt

European Patent Office

Office européen des brevets

(11) Publication number: **0 030 135 B2**

(12) **NEW EUROPEAN PATENT SPECIFICATION**

(45) Date of publication of the new patent specification: **23.01.91**

(51) Int. Cl.⁵: **A 61 N 1/36, H 03 K 17/90**

(21) Application number: **80304272.0**

(22) Date of filing: **27.11.80**

(54) Pacemaker with Hall effect element.

(30) Priority: **28.11.79 US 98168**
**28.11.79 US 98167**

(43) Date of publication of application:
**10.06.81 Bulletin 81/23**

(45) Publication of the grant of the patent:
**02.05.85 Bulletin 85/18**

(45) Mention of the opposition decision:
**23.01.91 Bulletin 91/04**

(84) Designated Contracting States:
**CH DE FR GB LI NL SE**

(56) References cited:
**EP-A-0 011 949**
**FR-A-1 445 090**
**GB-A- 985 797**
**GB-A-2 014 858**
**US-A-3 661 089**

(73) Proprietor: **MEDTRONIC, INC.**
**3055 Old Highway Eight P.O. Box 1453**
**Minneapolis Minnesota 55440 (US)**

(72) Inventor: **Thompson, David Lee**
**1660 Onondaga Street NE**
**Fridley Minnesota 55432 (US)**
Inventor: **Duggan, Stephen Randall**
**4180 120th Street West**
**Rosemount Minnesota 55068 (US)**
Inventor: **Roline, Glenn Milton**
**4118 - 211th Lane NW**
**Anoka Minnesota 55303 (US)**

(74) Representative: **Tomlinson, Kerry John et al**
**Frank B. Dehn & Co. European Patent Attorneys**
**Imperial House 15-19 Kingsway**
**London WC2B 6UZ (GB)**

EP 0 030 135 B2

Courier Press, Leamington Spa, England.

## Description

The invention in general relates to body tissue stimulators, and more particularly to a cardiac pacemaker having one or more characteristics that can be varied by means of a magnetically actuated switch.

The art of implantable cardiac pacemakers was first disclosed by Greatbatch in U.S. Patent No. 3,057,356 entitled "Medical Cardiac Pacemaker", which issued in 1962. The device disclosed by Greatbatch included a relaxation oscillator that generated electrical pulses at a fixed rate. These pulses were applied to the heart through a lead to cause the heart to contract each time a pulse occurred.

Early in the development of the art of cardiac pacemakers it became evident that it was desirable to design pacemakers so that the parameters of the output pulse could be varied. An early means for varying the output pulse incorporated a reed switch. See, for example, U.S. Patent No. 3,311,111 issued to D. L. Bowers and U.S. Patent No. 3,518,997 issued to R. W. Sessions. The reed switch is a pair of magnetically susceptible and conductive reeds which maintain themselves separated by spring action unless they are merged together by magnetic forces. Generally the magnetic forces are applied by means of a magnet held external to the body in which the pacemaker is implanted. Generally the closing of the reed switch by the magnet actuates circuitry within the pacemaker which alters the output parameters according to some predetermined scheme. By this means the output parameters of an implanted pacemaker can be adjusted externally of the body.

Many different schemes which incorporate a reed switch have been developed for adjusting the output parameters of an implanted pacemaker externally to the body. See for example U.S. Patent Nos. 3,623,486 issued to Baroug V. Berkovits; 3,631,860 issued to Michael Lopin; 3,738,369 issued to Theodore P. Adams and David L. Bowers; 3,805,796 issued to Reese S. Terry, Jr. and Gomer L. Davies; and 4,066,086 issued to Clifton A. Alferness and John M. Adams. The latter patent describes the method most commonly used, in which the reed switch is employed to actuate a circuit for receiving radio frequency programming signals. In this manner a wide variety of output parameters can be varied using a single switch. Because of the above developments, physicians have become accustomed to using magnets to vary output parameters of cardiac pacemakers.

Although the reed switch has become almost universal in pacemakers it is recognized as having several disadvantages. The reed switch is generally the only mechanical element in the pacemaker and for this reason it is more susceptible to damage and breakage than the electronic elements which make up the rest of the pacemaker. In addition, in order to fit into a pacemaker, the switch must be made very small which adds to its fragility. Generally, the tiny reeds of the switch are encased in a glass capsule for protection, but the glass capsule itself is susceptible to being fractured.

The modern pacemaker is generally wafer shaped so that it may be placed underneath the skin of the body with a minimum bulge. A characteristic of the reed switch is that, in order for it to respond to a magnetic field, it must be placed along the thin axis of the pacemaker, or at least have a component of its length along this axis. Generally, pacemakers are now thinner than the length of a typical reed switch so that incorporating a reed switch within the pacemaker is an orientation in which it operates reliably has become increasingly difficult. One attempt to overcome the disadvantages of a reed switch has been described in U.S. Patent No. 3,766,928 issued to Goldberg et al. This method incorporates a potentiometer which is affixed to a small diametrically magnetized disc magnet. A second magnet is rotated outside the body to cause the disc magnet to rotate and turn the potentiometer. This method itself has numerous disadvantages including the fact that it is also mechanical, and very small and thus prone to breakage. In addition, the manipulation of the second magnet in order to adjust the potentiometer is more difficult and complex than the manipulation of the magnet required to actuate a reed switch.

GB—A—985797 discloses a pacemaker with a timing element of variable resistance in the form of a Hall effect element which is affected by an external control magnet to give the required change in resistance.

According to the invention, there is provided a body-implantable pulse generator for providing stimulating pulses to living tissue, said pulse generator having a circuit capable of being activated by a magnetic field, characterised by said circuit comprising a Hall effect element, means for causing electric current to flow through said Hall effect element for time periods having a duration that is shorter than the time intervals between said periods, and latch means responsive to the output of the Hall effect element and arranged to maintain its output in one state between said time periods substantially while the magnetic field is applied to said Hall effect element. The Hall effect element may be activated by a magnetic field and is significantly smaller and more durable than a reed switch. The Hall effect element may operate effectively when oriented in a direction substantially parallel to the larger plane of the pacemaker; it enables the provision of a pacemaker which incorporate no mechanical parts.

Hall effect elements are described in "Magnetically Activated Monolithic Integrated Circuits For Analog and Digital Applications", by Robert A. Anaselmo and Michael J. Oppenheimer, technical publication 71—11 published by the Sprague Electric Company and available from Technical Literature Service, Sprague Electric Company, North Adams, Massachusetts 01247. Although magnetically activated monolithic integrated circuits have been commercially available for about ten years they have not, up to now, been incorporated into pacemaker technology. There are numerous reasons for this. Pacemakers necessarily must be small and in addition implanted pacemakers necessarily have a limited supply of electrical energy so the current source must be totally contained in the pacemaker. However, magnetically activated Hall devices, whether in monolithic integrated circuit form or in the other form, require complex supporting circuitry and draw large currents. Thus, Hall effect technology has generally been rejected for pacemaker purposes with the result that this technology is relatively unknown among those practised in the pacemaker art.

The cardiac pacemaker of the present invention has a circuit which produces timing signals characterized in that the duration of the timing signals is shorter than the intervals between the timing signals. For such timing signals the net "ON" time will be less than the "OFF" time of the signals. Thus, circuits which are controlled by the timing signals may have an "OFF" time that is relatively short compared to the "ON" time of the circuits. Thus the net current drain of the circuits may be substantially reduced.

Preferably the intervals are substantially equal to the intervals between the stimulating pulses provided by the pulse generator and the time periods when current flows through the Hall effect element are less than 200 μ sec.

Early in the development of the art of cardiac pacemaker oscillators having a period shorter than the pulse generating rate were incorporated into the pacemaker. See, for example, U.S. Patent No. 3,557,796 issued to John W. Keller, Jr. et al. Such oscillators produced timing signals which permitted precise control of the pacemaker operating parameters. For instance, an oscillator providing timing signals at the rate of 10,000 Hz permit the duration of the stimulating pulse to be controlled in increments of tenths of a millisecond.

Providing timing signals at large rates such as 10,000 Hz requires extensive use of the energy of the pacemaker because each time the timing pulse is provided an additional drain on the power source of the pulse generator occurs. Since the total energy available to the conventional implanted pulse generator is limited to that stored in the battery at the time of implantation, any additional drains on the power source necessarily reduces the life of the pulse generator. Thus it is desirable to minimize the drain on the power source in implantable pulse generators.

U.S. Patent No. 4,164,945 issued to Jerome T. Hartlaub discloses a pulse generator in which the drain on the power source is reduced, while at the same time permitting precise control of the output parameters, by including two oscillators in the pulse generator. One oscillator oscillating at a relatively high frequency is used to control output parameters such as the pulse width which require smaller timing increments for control, while a slower oscillator is used to control the basic pulse repetition rate.

In the prior art pacemakers it is a characteristic of timing signals that they are produced in oscillatory fashion, that is one signal follows immediately upon another in wavelike fashion. Thus, generally over a given time the "ON" time of such signals is equal to or greater than "OFF" time of these signals. This significant ratio of "ON" time to "OFF" time causes an unnecessary drain on the pacemaker power source in many instances.

An embodiment of the invention will now be described by way of example and with reference to the accompanying drawings, in which:

FIG. 1 is a block schematic diagram of the pacemaker circuitry for a preferred embodiment of the invention showing the various signals provided between the digital and analog portions of the circuit;

FIG. 2 shows the arrangement of Figs. 2a and 2b which in turn show, in block format, the digital circuitry portion of the invention;

FIG. 3 shows the Hall effect circuitry.

Description of the Preferred Embodiment

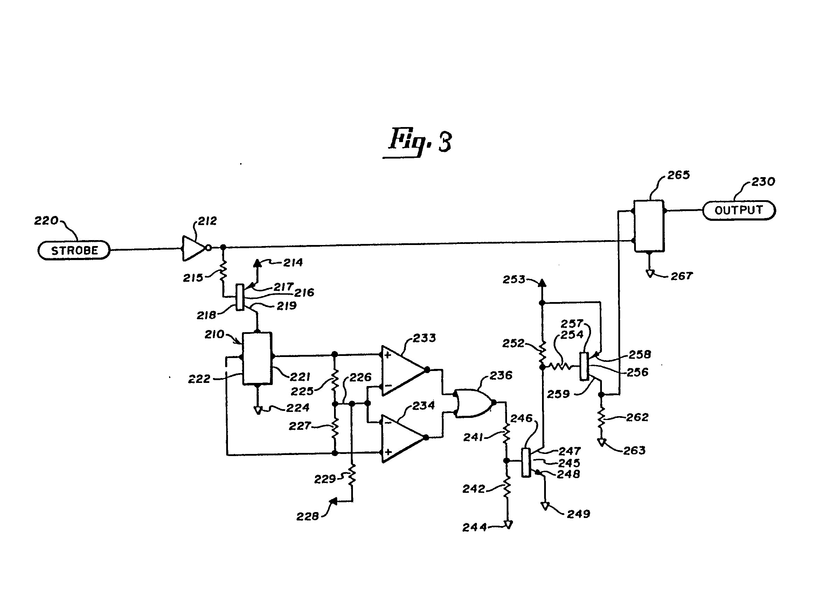

Referring now to Fig. 3 of the drawings, the circuitry for incorporating the Hall effect magnetic switch into a pacemaker is shown. The Hall effect element at 210 is preferably oriented on an integrated circuit chip within the pacemaker so that the plane of the switch (corresponding to the plane of the paper of the drawing) lies in the same direction as the broad plane of the pacemaker. A strobe pulse from the pacemaker circuitry is input at 220 and activated Hall element 210 for a short period at periodic intervals, preferably once each pacemaker pulse cycle. If the pacemaker has been placed in an auxiliary magnetic field in the conventional manner (see for example U.S. Patent No. 3,311,111 issued to D. L. Bowers) a positive voltage is output to the pacemaker circuitry at 230. This signal may be utilized within the pacemaker circuitry in a manner in which the signal generated by the closing of the conventional reed switch signal is utilized in the pacemaker; examples of apparatus and methods for utilization of the signal are discussed below, but these are not intended to be limiting.

In addition to the Hall effect element 210 the circuitry of Fig. 3 includes an inverter 212; resistors 215, 225, 227, 229, 241, 242, 252, 254 and 262; transistors 216, 245, and 256; operational amplifiers 233 and 234; an OR gate 236; and a d-type latch 265 (commonly referred to as a flip-flop). Connections to the positive voltage of the circuitry (also called the high or logic "1" voltage state) are shown by a solid triangle such as at 214. Connections to the ground state (also called the low or logic "0" state) of the circuitry are shown by open triangles such as at 224. Other than element 210, the circuit elements of Fig. 4 are all conventional and thus will not be described further.

The strobe pulse input at 220 is provided by the pacemaker circuitry which shall be described below. Strobe input 220 is applied to the input of inverter 212. The output of inverter 212 is applied to the clock input of latch 265 and to the base 218 of p-type transistor 216 through 20K resistor 215. The emitter 217 of transistor 216 is connected to the positive voltage source at 214, while its collector 219 is applied to one end of solid state switch element 210. The other end of the switch element 210 is connected to ground at 224. The sides of the switch element, which correspond to the switch terminals are connected to operational amplifiers 233 and 234. As shown in the drawing the left side is connected to the noninverting input of operational amplifier 234 while the right side is connected to the noninverting input of operational amplifier 233, although the choice of which side of the element 210 is connected to which of the operational amplifiers 233 and 234 is arbitrary. The inverting inputs of operational amplifiers 233 and 234 are connected to the positive voltage source at 228 through 22 megohm resistor 229. The inverting and noninverting inputs of operational amplifier 233 are connected through 2K resistor 225, and the inputs of operational amplifier 234 are connected through 10K resistor 227. The outputs of operational amplifiers 233 and 234 are applied as inputs to OR gate 236. The output of OR gate 236 is applied to the base of n-type transistor 245 through 10K transistor 241. The base 246 of transistor 245 is also connected to ground 244 through 10K resistor 242. The emitter 248 of transistor 245 is connected to ground at 249 while the collector 247 is connected to the positive voltage source at 253 through 10K resistor 252 and to the base of p-type transistor 256 through 10K resistor 254. The emitter 258 of transistor 256 is connected to the positive voltage source 253, while the collector 259 is connected to the data input of flip-flop 265 and to ground 263 through 100K resistor 262. The set and reset inputs of flip-flop 265 are connected to ground at 267. The "Q" ouput of flip-flop 265 is applied to output 230 to the pacemaker circuitry.

Solid state switch element 210 in the preferred embodiment is simply a piece of n-type silicon deposited on a ceramic chip along with other parts of the integrated circuitry of the pacemaker. Preferably the piece of silicon is approximately 30—40 mils (0.76—1.0 mm) square. If desired, a prefabricated Hall effect sensor element may be used, such as Microswitch No. 632SS4 manufactured by Honeywell, Inc. of Freeport, Illinois 61032.

The circuit just described operates in the following manner. The strobe pulse entering at 220 is generally a positive or a logic "1" signal of very short duration; in the preferred embodiment it is approximately 25 microseconds. The output of inverter 212 will thus be an approximately 25 microsecond negative pulse, which turns transistor 216 on causing a current to flow from voltage source 214 to ground 224 across switch element 210. The purpose of transistor 216 is to produce the appropriate current gain for driving the switch element 210. In the preferred embodiment the strobe output is rated at about 50 microamps whereas the solid state switch element 210 takes typically 5 to 10 milliamps, thus a current gain of 100 to 200 is required. Resistor 215 protects the emitter-base junction in a conventional manner.

If the Hall effect element 210 is an auxiliary magnetic field when the 25 μsec pulse of current flows across it, then a voltage proportional to the magnetic field is developed across sides 221 and 222 of element 210. The sign of the voltage will depend on whether the magnetic field is into or out of the plane of the drawing, that is whether the north or south pole of the magnet is held above the pacemaker. As will be discussed below, the embodiment of the circuits shown respond properly whether a voltage from right to left, or from left to right is developed. It is contemplated that in some form of the invention a distinction may be made between the sign of the voltage, or what amounts to the same thing, between whether a north pole or a south pole magnet is held above the pacemaker. That is, if one pole is used which develops one sign of a voltage the pulse signal may be employed to produce a first effect in the pacemaker, while if the opposite magnetic pole is used and the opposite sign developed a second effect may be produced in the pacemaker.

Operational amplifiers 233 and 234 and resistors 225, 227 and 229 are set up to operate as a pair of comparators. The middle line 226 to the inverting input of operational amplifiers 233 and 234 provides a reference voltage. If the magnetic field applied to element 210 is such that the right side of the element goes high, operational amplifier 233 turns on and the ouput goes to a high or logic "1" state; on the other hand, if the left side 222 of element 210 goes high then operational amplifier 234 turns on and its ouput goes to a logic "1". Thus, if element 210 is in the auxiliary magnetic field there will be a logic "1" signal on one of the inputs to OR gate 236 and therefore its output will become a logic "1".

The purpose of the circuit containing transistors 245 and 256 is to provide current gain and at the same time to invert the signal from OR gate 236 twice so that it will have a proper sign to produce the correct output at flip-flop 265. The logic "1" signal or OR gate 236 turns n-type transistor 245 on which puts a low or logic "0" signal on the base of p-type transistor 256, which in turn, turns that transistor on, and places a positive or logic "1" signal on the d input of flip-flop 265. This circuit may be eliminated in some embodiments of the invention. For example, if the Hall circuitry is implemented with CMOS type logic in an integrated circuit then the output of OR gate 236 will have a sufficient power level to trigger gate 265 without the need for power gain. Thus, in this case, the ouput of OR gate 236 may be applied directly to the data output of gate 265 and the invention will operate as described below.

If element 210 is in a magnetic field, the rise and fall of the signal on the d input of flip-flop 265 will follow the rise and fall of the strobe pulse with a slight delay. The delay is due to the time constant of the circuitry described above; that is, it takes a finite amount of time for each of the circuit elements such as operational amplifiers 233 and 234 and transistors 216, 245, and 256 to respond to signal at their inputs. Thus, when the inverter strobe signal from the output of inverter 212 is going from logic "0" to a logic "1" (the tail of the strobe pulse) the data input of flip-flop 265 will still be at a logic "1", and will remain so for the period of the delay through the circuit. The change of the pulse applied to the clock input of flip-flop 265 from logic "0" to logic "1" clocks the "Q" output of flip-flop 265 to the value of the data input, which is a logic "1". Since the set and reset inputs are held at logic "0" the "Q" output of flip-flop 265 remains at a logic "1" until the flip-flop is cocked while a logic "0" signal is applied to its data input. From the discussion above it is seen that this will happen on the first strobe pulse after the magnetic field is removed from the pacemaker and thus from element 210. Thus, the net effect of the invention is to provide a high or logic "1" signal at the output 230 substantially during the time period between the first strobe pulse after the magnetic field is placed on the pacemaker until the first strobe pulse after the magnetic field is removed from the pacemaker.

An important characteristic of the strobe signal is that the period of time in which the signal is "ON" is shorter than the intervals between signals. As discussed above, in the embodiment of the invention described, the strobe signal lasts for a period of 25 µsec and as will be described below the intervals between the strobe signals are of the order of 1/2 to 1 second. Preferably, the time period in which the strobe is on is less than 200 µsec; however, any period which is shorter than the time intervals between the strobe signals is usable.

In the circuit described above the solid state switch element 210 has about 250 nanoamps of average current drain. This current drain is very small compared to the total current drain of a conventional pacemaker, and thus the incorporation of the solid state magnetic switch circuit of the invention into the pacemaker does not substantially decrease the longevity of the pacemaker.

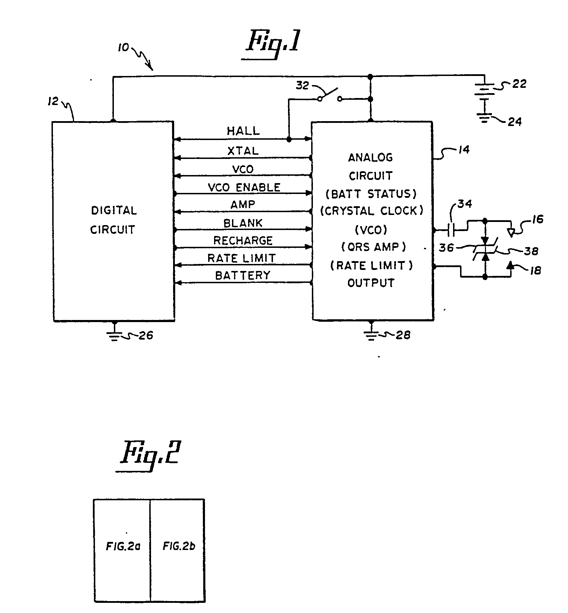

Turning now to the description of the pacemaker circuitry which produces the strobe pulse, Fig. 1 shows a schematic diagram of a cardiac pacemaker circuit according to an exemplary embodiment of the invention. The cardiac pacemaker 10 includes a digital circuit 12 which communicates with an analog circuit 14 to produce an electrical pulse which is delivered via outputs 16 and 18 to leads (not shown) for application to the heart (not shown) to cause the heart to contract. The leads through which the pulses are applied to the heart, the type of pulses applied, and the response of the heart to the pulses is well known in the art and will not be discussed herein.

The embodiment of the pulse generator 10 which shall be described is of the type that is implantable within the human body.

The analog circuit as shown in Fig. 1 consists of a number of generally separate electrical systems. These systems include a battery status monitor, a crystal oscillator clock, a voltage control oscillator clock, a QRS sensing amplifier, a pulse rate limiting circuit and output circuitry which includes a voltage doubler. Each of these analog systems are well known in the art and will not be discussed in detail herein. For a complete description of certain of these circuits reference is made to the following patent applications, commonly owned with the present application: European Applications 79302447.2 (0011931) and 79302450.6 (0011934).

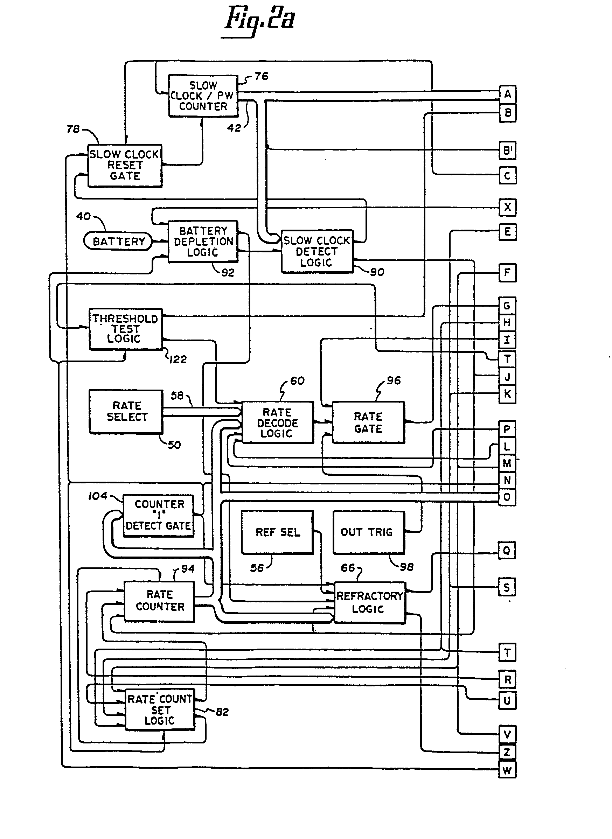

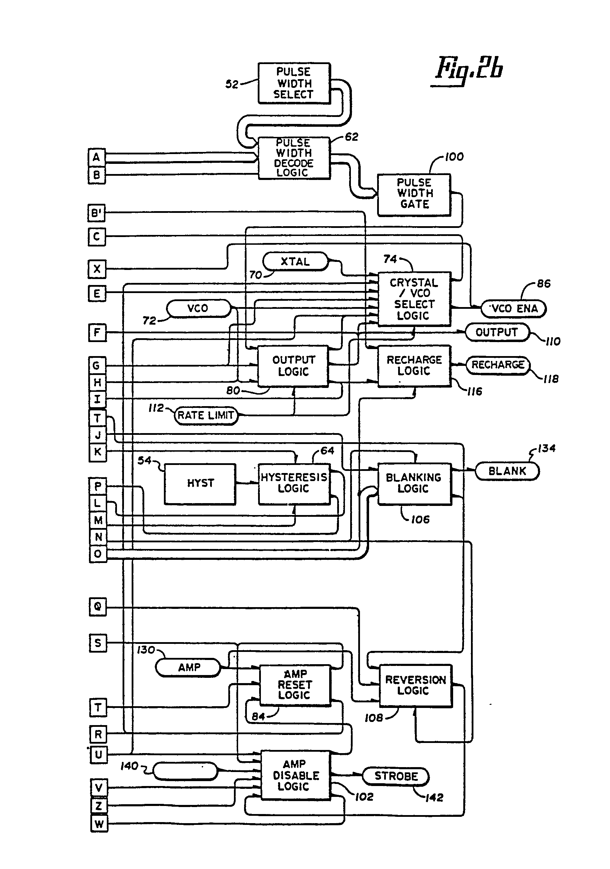

The digital circuit 12 includes the mode selection circuitry, the digital logic necessary to produce each of the selectable output modes, and digital timing means for causing pulses to be generated from pulse generator 10 in the selected manner. A more detailed description of digital circuit 12 is given below in the discussion relating to Figs. 2a and 2b.

Both the digital and analog circuits are powered by battery 22, which may be a conventional lithium-iodide battery generating +V, or approximately 2.8 volts, connected between a source of reference potential, such as ground 24, and the circuits 12 and 14. In the present description, a signal at the power supply voltage may be referred to alternatively as a logic "1" or a high signal. A signal at the ground voltage may be referred to alternatively as a logic "0" or a low signal. The digital circuit 12 is connected to ground at 26 and the analog circuit 14 is connected to ground at 28. Switch 32 is the Hall effect switch described above with reference to Fig. 4 and may be "closed" by placing a magnet (not shown) in close proximity to pulse generator 10 in a manner well known in the art. When HALL EFFECT switch 32 is "closed" a +V volts or a logic "1" HALL signal is applied both to digital circuit 12 and analog circuit 14. When the magnet is removed from the vicinity of the pulse generator 10 HALL switch 32 "opens" and a ground, or logic "0" signal is applied to digital circuit 12 and analog circuit 14. The manner in which such signals are applied shall be described in more detail below.

As mentioned above, outputs 16 and 18 communicate with conventional cardiac pacemaker leads to apply the pulse generator signal to the heart. Output 18 may consist of the outer metal casing of pulse generator 10 or it may connect with an electrode wire within a lead system, depending upon the type of cardiac pacemaker lead selected. Output 16 is coupled to analog circuit 14 through a capacitor 34 to prevent DC leakage in the case of some system failures. A pair of diodes 36 and 38 have their anodes coupled together and their cathodes coupled to ouputs 16 and 18 respectively. Diodes 36 and 38 function in the conventional manner to prevent damage to the circuitry of pulse generator 10 in the case of large extraneous signals, such as may be caused by electrocautery.

Analog circuit 14 provies the XTAL, VCO, AMP, RATE LIMIT and BATTERY signals to digital circuit 12. Digital circuit 12 provides the VCO, ENABLE, BLANK, and RECHARGE signals to analog circuit 14.

The XTAL signal is a square-wave pulse signal occurring at a frequency of 32,768 Hz and the VCO is a square-wave pulse signal having a frequency of 20,000 Hz whenever the voltage of battery 22 is equal to 2.8 volts and decreases approximately proportional to the square of the voltage to about 10,000 Hz when the battery voltage is equal to 2.0 volts. This decrease of the VCO frequency is used, as shall be explained in more detail below, a provide an increase in the pulse width as the voltage from battery 22 decreases, in order to maintain a constant energy of the pulse.

The VCO ENABLE signal provided from digital circuit 12 to analog circuit 14 is normally a logic "1". However, at the time the stimulation pulse is to be provided, the VCO ENABLE signal becomes a logic "0" and the VCO is enabled to begin providing pulses. The VCO ENABLE signal remains logic "0" until after the stimulating pulse has been provided, at which time it returns to logic "1" and the VCO becomes disabled.

The AMP signal is provided from the output of the analog QRS sensing amplifier and is normally a logic "1" signal. Each time the QRS amplifier senses a naturally occurring QRS signal, it becomes a logic "0" pulse signal.

The BLANK signal provided from digital circuit 12 is normally a logic "1" which becomes logic "0" for approximately 100 msec following the provision of a stimulating pulse from pulse generator 10 of the sensing of a natural heartbeat. The BLANK signal is used to prevent the QRS sensing amplifier within analog circuit 14 from sensing any signals during the 10 msec time interval and to allow the components within the sensing amplifier circuit to reset themselves after sensing a signal.

The RECHARGE signal is a normally logic "0" which becomes logic "1" for approximately 10 msec after the stimulating pulse has been provided by generator 10. The purpose of the RECHARGE signal is to close a switch in analog circuit 10 to allow an ouput capacitor to recharge after every output pulse.

The RATE LIMIT signal which is provided from analog circuit 14 to digital circuit 12 is a normally logic "0" signal which becomes logic "1" after the provision of the stimulation pulse for approximately 500 msec to set an upper rate limit of approximately 120 pulses per minute for pulse generator 10. This rate limitation function is used as a backup and safety feature against the possibility of an integrated circuit of crystal oscillator failure that may cause high rate output to occur.

The BATTERY signal applied from analog circuit 14 to digital circuit 12 is a logic "1" signal so long as the voltage provided from battery 22 is above a certain minimum level, for example 2.0 volts, and is a logic "0" signal whenever the voltage from battery 22 falls below the minimum level.

Referring now to Fig. 2, there is shown the manner of arranging Figs. 2a and 2b to form a block diagram of digital circuit 12. In Figs. 2a and 2b any signals which are received from, or applied to analog circuit 14 are designated by the particular label for the signal placed within an oval, for example the battery signal just discussed above is shown at 40 in Fig. 2a. The oval labeled "strobe" is an output of the strobe input 220 in Fig. 3 and to other portions of the digital circuit which shall be described below. All provisions of power supply voltage or ground coupled to each block have been deleted, although it should be understood that these signals are necessary and should be coupled in a known and accepted manner of designing digital logic circuits. For each of the blocks shown in Figs. 2a and 2b data signals are shown as being applied to the left side of the block, reset signals are shown as being applied to the bottom of the block, set signals are shown as being applied to the top of the block, and output signals are shown as originating from the right side of the block. Arrows pointing into the block indicate a signal applied to the block, whereas arrows pointing out of the block designate a signal originating from the block. Wherever a plurality of lines are transmitted from, or to a particular block circuit, such as a parallel output from a counter or shift register such plurality of lines are represented as wide lines such as at 42 in Fig. 2a.

Turning now to a description of the manner in which the various subcircuits of the pacemaker are connected and interact as shown in Figs. 2a and 2b, the pulse rate and hysteresis rate selection circuitry is shown at 50, the pulse width selection circuit is shown at 52, the hysteresis function selection circuit is shown at 54, and the refractory period selection circuit is shown at 56. Eleven pulse rates and two hysteresis rates are selectable. Selecting a rate, as shall be described below, causes a predetermined combination of logic "1" and logic "0" signals to be output from rate selection circuitry 50 and applied to rate decode logic circuitry 60. If the hysteresis function is selected a logic "1" signal is output from hysteresis function selection circuitry 54 and applied to hysteresis logic 64; if the nonhysteresis function is selected a logic "0" signal is output and applied between the same circuits. The selection of the pulse width causes a predetermined set of logic "0" and logic "1" signals to be output from the pulse width selection circuitry 52 and applied to the pulse width decode logic circuitry 62. Selection of the refractory period causes a predetermined one of either a logic "0" or a logic "1" signal to be ouput from refractory selection circuitry 56 and applied to refractory logic 66.

The timing of the pulse generator function is provided by the XTAL oscillator and VCO oscillator signal originating from analog circuit outputs 70 and 72 respectively, and having the frequencies described above. The XTAL and VCO outputs 70 and 72 are applied to crystal/VCO select logic 74. The VCO output also communicates directly with output logic 80, rate count set logic 82, and amp reset logic 84. Crystal/VCO select logic 74 selects one of the XTAL or VCO signals and provides it as the upper output signal which is applied to slow clock/PW counter 76 and to the set input of the slow clock reset gate 78. If any of the inputs to crystal/VCO select logic 74 (other than the XTAL and VCO inputs) are a logic "1" the circuitry selects the VCO signal, and at the same time applies a logic "0" signal through its lower output to VCO ENABLE output 86. If all of the inputs (other than the XTAL and VCO inputs) are a logic "0" the XTAL signal is selected and a logic "1" signal is output to VCO ENABLE 86. The upper output of the crystal/VCO select logic, either XTAL or VCO, provides a fast clock signal for the pulse generator circuit. The VCO pulse starts low (logic "0") and is triggered by the XTAL clock pulse with the result that there is an apparent synchronization between the XTAL and VCO clocks.

Slow clock/PW counter 76 is an eightstage binary counter connected in a known manner. The ouput signal from slow clock 76 is provided to slow clock detect logic 90, to recharge logic 116 and to pulse width decode logic 62. When slow clock/PW counter 76 counts 179 fast clock cycles detect logic 90 reads this count and provides an output signal through its upper output to slow clock reset gate 78, which in turn provides an output signal to reset slow clock/PW counter 76. The resetting of the slow clock/PW counter 76 after each 179 fast clock cycles determines a time interval called slow clock which is equal to approximately 5.49 msec.

Slow clock detect logic 90 provides a slow clock signal through its lower output to rate counter 94, which is an eightstage binary counter connected in a known manner. The output of rate counter 94 is provided to rate decode logic 60, counter "1" detect gate 104, refractory logic 66, blanking logic 106, and recharge logic 116. The count of rate counter 94 is read by rate decode logic 60 and when the number of counts determined by the inputs of rate decode logic from rate select 50 and hysteresis logic 64 is reached, rate decode logic 60 provides an output signal to rate gate 96. The output from rate gate 96 is provided to crystal/ VCO select logic 74, in order to turn on the VCO oscillator as described above, and to output logic 80 in order to initiate the pulse output sequence as shall be described below. Output trigger 98 is a test pin by means of which an external output can be applied to rate gate 96 to force the pulse generator into rate limit either to test the rate limit circuitry and/or in order to perform a laser trim of a resistor on the rate limit circuitry at the time of manufacture.

Output logic 80 is responsive to signals from the pulse width gate 100, and the outputs of rate gate 96 and VCO output 72. The reset input of output logic 80 is responsive to the rate limit output 112 from analog circuit 14. The upper output of output logic 80 is applied to crystal/VCO select logic 74, to rate count set logic 82 and to amp disable logic 102. The middle output is applied to crystal/VCO select logic 74, to output 110 to the analog circuit, to rate count set logic 82, to amp disable logic 102 and to the reset input of hysteresis logic 64. The lower output is applied to recharge logic 116 and rate gate 96.

As mentioned above the signal from rate gate 96 activates the VCO oscillator. On the first VCO pulse output logic 80 is clocked and its upper output changes from logic "0" to logic "1". This signal is applied to crystal/VCO select logic 74 to shut off the VCO oscillator during the period in which the pacemaker is in rate limit as shall be discussed further below, and clocks rate count set logic 82 so that its lower output goes to logic "1". This signal is applied to the set input of rate counter 94 to force all its stages to a logic "1" output state. Counter "1" detect gate 104 detects this "all 1's" conditions and changes its output from logic "0" to a logic "1". The signal from counter "1" detect gate is applied to slow clock reset gate 78 to reset slow clock/PW counter 76 and to the reset input of rate count set logic 82.

On the next VCO oscillation output logic 80 is again clocked and its middle output changes from logic "0" to logic "1". The application of this signal to output 110 initiates the pulse generator output in analog circuit 14. The same signal applied to crystal/VCO select logic 74 continues to enable the VCO oscillator for the duration of the output pulse. The signal is also applied to rate count set logic 82 to enable it so that on the next VCO oscillation its upper output goes from logic "0" to logic "1". This signal is in turn applied to rate counter 94 and clocks it to an overflow condition removing the logic "1" status of each of its stages to prepare the rate counter for the next count cycle.

Rate limit output 112 from analog circuit 14 is applied to the reset input of output logic 80 and to crystal/VCO select logic 74. The rate limit function is a secondary one-shot function on a separate die and is used as a backup against the possibility of integrated circuit or crystal oscillator failure which might create high rate output. The signal applied from rate limit output 112 is normally a logic "0", but during an approximately 500 msec period following the output pulse it is a logic "1". The logic "1" signal applied to the reset input of output logic 80 holds the middle output at a logic "0" until the end of the rate limit period, thereby blocking the output pulse during this period. Thus the rate limit output inhibits rates faster than approximately 120 beats per minute and does not allow the output to go to a 2:1, 3:1, etc. divide condition. The circuitry of output logic 80 is such that if an output pulse were to be called for before the rate limit time period had elapsed, the circuitry is held reset. Then, on the next positive clock transition after the rate limit interval runs out and the signal from rate limit 112 returns to logic "0" the output pulse is initiated.

The signal from rate limit 112 applied to crystal/ VCO select logic 74 shuts off the VCO oscillator during the period in which the upper output logic 80 is logic "1" and the rate limit circuitry is on. Thus, if the pacemaker is in a runaway condition in which the output pulse is being called for during the rate limit period, the VCO oscillator is held in the OFF condition. This function minimizes current drain if a runaway condition should arise.

As discussed above, just prior to the initiation of the output pulse the rate counter is set to an "all 1's" condition which condition is detected by counter "1" detect gate 104 which thereupon signals slow clock reset gate 78 to reset slow clock/PW counter 76. Slow clock/PW counter 70 then begins increasing its count again in response to VCO oscillator pulses from crystal/VCO select 74. Pulse width decode logic 62 reads the count of slow clock/PW counter 76, and when it has reached a count determined by the output of pulse width select 52, pulse width decode logic 62 provides a signal to pulse width gate 100. The output of pulse width gate 100 is provided to output logic 80 to enable the output circuitry so that the output pulse is terminated on the next VCO clock cycle.

The lower output of output logic 80 changes from logic "0" to logic "1" at the termination of the output pulse. The signal is applied to the recharge circuitry to enable it so that after a period determined by a signal provided from slow clock/PW counter 76 the output from recharge logic 116 goes from logic "0" to logic "1" to initiate the recharge interval. The signal is applied to the analog circuit through recharge output 118. Approximately 10 msec after the initiation of the recharge interval logic 116 is reset via a pulse from rate counter 94 and thereby resetting the output to logic "0" to terminate the recharge interval.

Battery output 40 from the analog circuit 14 is provided to battery depletion logic 92. Battery depletion logic 90 is also responsive to the lower output of amp disable logic 102 and the lower output of crystal/VCO select logic 74. The upper output of battery depletion logic 92 is provided to refractory logic 66 and the lower output is provided to slow clock detect logic 90. Battery depletion logic 92 is clocked by crystal/VCO select logic 74 each time the VCO is enabled just prior to an output pulse. This timing of the clock signal prevents supply ripple during or for a period after the pulse from prematurely triggering the battery depletion condition. If the battery is depleted, battery depletion logic 92 provides a logic "0" signal at its lower output which is applied to slow clock detect logic 90 to extend the slow clock interval by 11.1 percent to provide a 10 percent slowdown in the pulse rate. At the same time, a logic "1" signal is provided to refractory logic 66, from the lower output of battery depletion logic 92 to ensure a stable refractory interval described below. The battery depletion logic circuit is latchable) i.e. if the battery signal shows depletion at the time of any clock pulse provided by crystal/VCO select logic 74, then any subsequent clock pulse results in the output which extends the slow clock interval. The logic may be "unlatched" by the signal from amp disable logic 102, which is provided whenever Hall effect switch 32 is closed. If the BATTERY signal no longer is indicating battery depletion at the time that the Hall effect switch is closed then the battery depletion logic resets to its normal output. However, if the BATTERY signal indicates battery depletion at the time that the Hall effect switch is closed then battery depletion logic 92 remains latched.

We now turn to a discussion of the pacemaker circuitry related to the demand function of the pulse generator, i.e. the function that prevents a generated pulse from being output if a natural heartbeat is detected. Amp output 130 from analog circuit 14 is provided as one input to amp reset logic 84. Other inputs to amp reset logic 84 are from VCO output 72 and the upper output of amp disable logic 102. The upper output of amp reset logic 84 is applied as an input to rate count set logic 82, amp disable logic 102, crystal/VCO select logic 74, and as the set input to hysteresis logic 64. The lower output is provided as an input to rate counter 94 and crystal/VCO select logic 74. In normal operation of the pacemaker a detected heartbeat causes a clock signal to be applied to amp reset logic 84 from output 130. If the upper output of amp disable logic 102 is a logic "1" the clock signal from output 130 causes the upper output of amp reset logic 84 to change to a logic "1" state. The logic "1" signal applied to rate count set logic 82 causes rate counter 94 to be set to "all 1's" as described above and the signal to crystal/VCO select logic 74 returns the VCO on. On the first VCO pulse amp reset logic 84 is clocked to provide a logic "1" signal at its second output which is applied to rate counter 94 and crystal/VCO select logic 74. The signal to the rate counter 94 clocks the rate count stages to an overflow condition, while the signal to crystal/VCO select logic 74 maintains the VCO ON condition. Amp reset logic 84 resets itself at the same time so that the upper output goes to a logic "0" and one VCO pulse later the second output goes to a logic "0" thereby turning off the VCO and readying the amp reset logic for the next cycle triggered by the next natural heartbeat.

Blanking logic 106 is responsive to the lower output of slow clock detect logic 90 and the output of rate counter 94. Its set input is responsive to the output of counter "1" detect gate 104. The upper output of blanking logic 106 is provided to blank output 134 and the lower output is provided to reversion logic 108 an threshold logic 122. Blanking logic 106 is clocked each time the lower output of slow clock detect logic 90 changes from logic "0" to logic "1". Most of these clock pulses have no effect on the output states of blanking logic 106 because the circuitry has a self-latching loop that causes the output to retain their previous state with each clock pulse. This state is normally a logic "1" for the upper output and also a logic "1" for the lower output. The signal from counter "1" detect gate 104 however sets blanking logic 106 at the beginning of the output pulse and after a natural heartbeat is sensed. This changes the upper output to a logic "0" to initiate the blanking period and also changes the lower output to a logic "0". The SET signal from counter "1" detect gate 104 goes to a logic "0" on the next VCO pulse, however the blanking logic 106 maintains itself latched in the blanking output condition until approximately 100 msec after the initiation of the pulse when the signal from rate counter 94 relatches blanking logic 106 into the "nonblanking" state described above, thereby terminating the blanking interval. During the blanking interval the sensing amplifier in analog circuit 14 is blanked thereby preventing any input to amp reset logic 84 from amp output 130.

Amp disable logic 102 is responsive to the outputs of Hall Circuit input 140 and reversion logic 108, the lower output of refractory logic 66, the upper output of amp reset logic 84, and the middle and upper outputs of output logic 80. Hall circuit input 140 carries the signal from output 230 of Fig. 3. The lower output of amp disable logic 102 is provided to threshold test logic 122 and battery depletion logic 92. The upper output is provided to amp reset logic 84, while the middle output provides a strobe output 142 which is used elsewhere in the digital circuit and shall be described below. The output to strobe 142 is a logic "0" except in two instances: it is a logic "1" in the time period between the two output pulses from output logic 80, a time period of approximately 25 sec just at the initiation of the output pulse; it is also a logic "1" during the time in which the upper output from amp reset logic 84 is a logic "1", that is the period during which the rate counter is being set after each detected natural heartbeat, again a very short period. The lower output of amp disable logic 102 is normally a logic "1" but goes to a logic "0" during a time period between the trailing edge of the first strobe pulse after the Hall Effect switch 32 is closed until the trailing edge of the first strobe pulse after Hall Effect switch 32 is opened. The upper output of amp disable logic 102 is normally logic "1' whenever either the lower output from refractory logic 55 or the output from reversion logic 108 is a logic "0". The upper output is also a logic "0" during the period described above when the lower output is "0", that is during the approximate time period during which the Hall Effect switch is closed.

Refractory logic 66 is responsive to the output of refractory select 56, the upper output of battery depletion logic 92, the lower output of slow clock detect logic 90, and the output of the rate counter 94. The set input of refractory logic 66 is responsive to the output of counter '11' detect gate 104. The upper output of refractory logic 66 is applied to reversion logic 108, while the lower output is applied to amp disable logic 102. The output of counter "1" detect gate 104 goes to logic "1" just prior to the initiation of the output pulse, and just after the detection of a natural heartbeat as described above. This logic "1" signal sets refractory logic 66 causing its upper output to go to logic "1" and its lower output to go to logic "0". As can be seen from a discussion of the amp disable logic 102 and amp reset logic 84 above the logic "0" state of the lower output of refractory logic 66 causes the upper output of amp disable logic to go to a logic "0" which in turn disables amp reset logic 84 from activation of rate count set logic 82. When the rate counter reaches the count which determines the refractory period the input to refractory logic 66 from rate counter 94 goes to a logic "1". The trailing edge of the same slow clock pulse from slow clock detect logic 90 that advanced rate counter 94 to the refractory count, clocks refractory logic 66 so that its upper output goes to a logic "0" while its lower output goes to a logic "1". This terminates the refractory period and allows the amp reset function to operate as discussed above. If the output of refractory select 56 is logic "0" a 325 msec refractory period is selected; if the output is a logic "1", a 400 msec refractory period is selected. As mentioned above, the input to refractory logic 66 from battery depletion logic 92 causes the refractory logic 66 to recognize an earlier count from rate counter 94 in order that the 11 percent slowdown of the slow clock and rate counter after the battery is depleted is offset and a stable refractory interval is obtained.

Reversion logic 108 is responsive to the lower output of blanking logic 106, the upper output of refractory logic 66, and the output of amp output 130. The output of counter "1" detect gate 104 is applied to the reset input of reversion logic 108. The output of reversion logic 108 is applied to amp disable logic 102 as discussed above. The logic "1" signal from counter "1" detect gate 104 resets reversion logic 108 just prior to the initiation of the output pulse or just after a natural heartbeat. The reset signal places a logic "1" signal on the reversion logic 108 output. At the same time the upper output of refractory logic 66 has become a logic "1" also. At the end of a 100 msec blanking interval the lower output of blanking logic 106 becomes a logic "1" as discussed above. With the inputs from both refractory logic and blanking logic being a logic "1" reversion logic 108 is enabled to receive inputs from amp output 130. This enabling period ends when the upper output of refractory logic 66 goes to a logic "0" at the end of the refractory interval. If two pulses from the amp output 130 are received during this reversion interval the output of reversion logic 108 goes to a logic "0" disabling the amp reset logic 84 through amp disable logic 102 as discussed above with respect to the refractory function. Since the refractory interval is either 325 or 400 msec the reversion interval is either 225 or 300 msec; therefore the fact that the reversion is effective if two pulses are detected means that any continuous signal of frequency greater than 8.8 Hz or 6.6 Hz (depending upon the refractory period chosen) is rejected by the reversion circuit.

Hysteresis logic 64 is responsive to the output of the hysteresis function select circuit 54, has a set input from the upper output of amp reset logic 84, and has a reset input from the middle output of output logic 86. Both the upper and lower outputs of hysteresis logic 64 are applied to rate decode logic 60. As discussed above, the amp output 130 signals a detected heartbeat the upper output of amp reset logic 84 goes to a logic "1". This signal sets hysteresis logic 64 so that the upper output goes to a logic "1" while the lower output goes to logic "0". The logic "1' signal is applied to rate decode logic 60 to enable the hysteresis rate. The logic "0" output is applied to rate decode logic 60 to disable all possible pacemaker rates except the two hysteresis rates. Hysteresis logic 64 will remain in this state as long as the heartbeat rate remains above the hysteresis rate, since each natural heartbeat resets rate counter 94 so that the hysteresis count is not reached. If the heartbeat drops below the hysteresis rate the counter reaches the hysteresis count and signals for an output pulse through rate gate 96 as described above. When the output pulse is initiated by the logic "1" signal from the middle output of output logic 80, a logic "1" reset signal is applied to hysteresis logic 64. The signal causes the upper output of hysteresis logic 64 to change the logic "0" state and the lower output to change to a logic "1" state. The logic "0" signal disables the hysteresis rate in rate decode logic 60 while the logic "1' signal enables the "normal" rates in rate decode logic 60 to provide an output signal at the selected rate. This continues until a natural heartbeat is again detected and the cycle repeats. If the output from hysteresis function select 54 is a logic "0" the upper output of hysteresis logic 64 will remain at a logic "0" state while the lower output will remain at a logic "1" state, which disables the hysteresis function. If the output of hysteresis function select 54 is a logic "1" the hysteresis function is enabled and hysteresis logic 64 operates as discussed.

Threshold test logic 122 is responsive to the lower output of blanking logic 106. The reset input of threshold test logic 122 is responsive to the lower output of amp disable logic 102. The upper output of threshold test logic 122 is applied to pulse width decode logic 62 while the lower output is applied to rate decode logic 60. As discussed above the lower output of amp disable logic 102 is normally a logic "1" which signal holds threshold test logic reset. Both outputs are held in the logic "0" state in this situation. The closing of the Hall effect switch 32 places a logic "0" signal on the lower output of amp disable logic 102 coinciding with the trailing edge of the strobe pulse which comes just at the initiation of the output pulse. This signal enables threshold test logic 122. There is no immediate effect on the rest of the pulse generator circuit. A normal output pulse or natural heartbeat (whichever is dictated by the circumstances) occurs. At the end of the 100 msec blanking period following either the normal output pulse or natural heartbeat the lower output of blanking logic 106 changes from a logic "0" to a logic "1". This signal clocks threshold test logic 122 which changes the lower output to a logic "1", while the upper output remains at a logic "0". The logic "1" signal is applied to rate decode logic 60 to enable the 100 pulse per minute rate. The next output pulse therefore occurs in 600 msec, unless the pacemaker rate or the natural heartbeat rate is faster than 100 beats per minute, in which case a pulse or natural heartbeat at this faster rate occurs. After this pulse or heartbeat threshold test logic 122 is again clocked by blanking logic 106. This third clocking again places the lower output in a logic "1" state and also places the upper output in a logic "1" state. The signal from the lower output again stimulates a 600 msec pulse interval, while the signal from the upper output is applied to pulse width decode logic 62 to cause the pulse width to be seventy-five percent of the selected output pulse width. Thus, on closing the Hall effect switch two normal output pulses at a rate of 100 beats per minute or higher are observed followed by a third pulse at the 100 pulse per minute rate, which pulse is seventy-five percent of the "normal" pulse width. After the narrower pulse threshold test logic 122 is again clocked by blanking logic 106, which signal causes the circuit to reset itself and hold itself reset until the Hall effect is again closed.

The above description of the invention has been in reference to a particular embodiment. It is evident that those skilled in the art can make numerous uses of, modifications of, and departures from the specific embodiment described herein without departing from the inventive concepts. For example, the Hall effect depends only on the flow of a charge in a magnetic field, thus conductive materials other than silicon may be employed in producing the Hall effect element 210. Various electronic parts of the circuit such as the resistors, transistors, operational amplifiers, and flip-flops may be replaced by equivalent electronic parts. Many other variations may be described.

## Claims

1. A body-implantable pulse generator (10) for providing stimulating pulses to living tissue, said pulse generator having a circuit capable of being activated by a magnetic field, characterised by said circuit comprising a Hall effect element (210), means (220, 212, 218) for causing electric current to flow through said Hall effect element for time periods having a duration that is shorter than the time intervals between said periods, and latch means (265) responsive to the output of the Hall effect element and arranged to maintain its output in one state between said time periods substantially while the magnetic field is applied to said Hall effect element.

2. A pulse generator as claimed in claim 1 having at least one broad surface and at least one narrow surface and wherein said Hall effect element is oriented in a plane substantially parallel to said broad surface.

3. A pulse generator as claimed in claim 1 or 2 further comprising a pair of comparators (233, 234) coupled across the Hall effect element such that one of the comparators produces an output signal when the Hall effect element is in a magnetic field and current flows through the Hall effect element.

4. A pulse generator as claimed in claim 3 including an OR gate (236) connected to receive the outputs of said comparators and arranged to produce an output signal when the Hall effect element is in a magnetic field and current flows through the Hall effect element, irrespective of the polarity of the magnetic field.

5. A pulse generator as claimed in claim 4 wherein said latch means is coupled to the output of said OR gate.

6. A pulse generator as claimed in any preceding claim wherein said duration is 200 microseconds or less.

7. A pulse generator as claimed in any preceding claim wherein said intervals are greater than 0.1 seconds.

8. A pulse generator as claimed in any preceding claim wherein said intervals are substantially equal to the intervals between said stimulating pulses.

9. A pulse generator as claimed in claim 8 including an oscillator with a period which is small relative to the interval between stimulating pulses, an output circuit for providing stimulating pulses, and an amplifier circuit for sensing natural heartbeats, and including means for producing timing signals responsive to said output circuit and said amplifier circuit to produce a timing signal of duration related to the period of said oscillator when a natural heartbeat is sensed or when a stimulating output pulse is provided.

**Patentansprüche**

1. Körperimplantierbarer Impulsgenerator (10) zur Anlieferung von Reizimpulsen an lebendes Gewebe, wobei der Impulsgenerator einen mittels eines magnetischen Feldes aktivierbaren Stromkreis aufweist, dadurch gekennzeichnet, daß dieser Stromkreis versehen ist mit einem Halleffektelement (210), mit einer Anordnung (220, 212, 218), die elektrischen Strom durch das Halleffektelement für Zeitperioden fließen läßt, die eine kürzere Dauer als die Zeitintervalle zwischen den Perioden haben, und mit einer auf das Ausgangssinal des Halleffektelements ansprechenden Latchanordnung (265), die so angeordnet ist, daß sie ihren Ausgang zwischen den Zeitperioden in einem Zustand hält, im wesentlichen während das magnetische Feld an das Halleffektelement angelegt wird.

2. Impulsgenerator nach Anspruch 1 mit mindestens einer breiten Oberfläche und mindestens einer schmalen Oberfläche, wobei das Halleffektelement in einer zu der breiten Oberfläche im wesentlichen parallelen Ebene ausgerichtet ist.

3. Impulsgenerator nach Anspruch 1 oder 2 mit zwei Komparatoren (233, 234), die derart an das Halleffektelement angekoppelt sind, daß einer der Komparatoren ein Ausgangssignal erzeugt, wenn das Halleffektelement in einem magnetischen Feld liegt und durch das Halleffektelement Strom fließt.

4. Impulsgenerator nach Anspruch 3 mit einer ODER-Schaltung (236) zur Aufnahme der Ausgangssignale der Komparatoren, die derart angeordnet ist, daß sie ein Ausgangssignal erzeugt, wenn das Halleffektelement in einem magnetischen Feld liegt und Strom durch das Halleffektelement fließt, unabhängig von der Polarität des magnetischen Feldes.

5. Impulsgenerator nach Anspruch 4, wobei die Latchanordnung an den Ausgang der ODER-Schaltung angekoppelt ist.

6. Impulsgenerator nach einem der vorhergehenden Ansprüche, wobei die Dauer 200 Mikrosekunden oder weniger beträgt.

7. Impulsgenerator nach einem der vorhergehenden Ansprüche, wobei die Intervalle größer als 0,1 Sekunden sind.

8. Impulsgenerator nach einem der vorhergehenden Ansprüche, wobei die Intervalle im wesentlichen gleich den Intervallen zwischen den Reizipulsen sind.

9. Impulsgenerator nach Anspruch 8 mit einem Oszillator, der eine mit Bezug auf das Intervall zwischen Reizimpulsen kleine Periode hat, einer Ausgangsschaltung zur Bereitstellung von Reizimpulsen, und einer Verstärkerschaltung zum Wahrnehmen von natürlichen Herzschlägen, sowie mit Mittels zum Erzeugen von Zeitsignalen in Abhängigkeit von der Ausgangsschaltung und der Verstärkerschaltung zwecks Erzeugung eines Zeitsignals mit einer mit der Periode des Oszillators verknüpften Dauer, wenn ein natürlicher Herzschlag wahrgenommen wird oder wenn ein Reizausgangsbereitgestellt wird.

**Revendications**

1. Générateur d'impulsions (10) implantable dans le corps, destiné à produire des impulsions de stimulation pour des tissus vivants, ledit générateur d'impulsions comportant un circuit capable d'être actionné par un champ magnétique, caractérisé en ce que ledit circuit comprend un élément à effet Hall (210), un dispositif (220, 212, 218) pour provoquer la circulation d'un courant électrique dans ledit élément à effet Hall pendant des périodes ayant une durée plus courtes que celles des intervalles de temps entre lesdites périodes et un circuit bistable (265) sensible à la sortie de l'élément à effet Hall et agencé pour maintenir sa sortie à un état entre lesdites périodes pratiquement pendant que le champ magnétique est appliqué audit élément à effet Hall.

2. Générateur d'impulsions selon la revendication 1, comprenant au moins une surface large et au moins une surface étroite et dans lequel ledit élément à effet Hall est orienté dans un plan pratiquement parallèle à ladite surface large.

3. Générateur d'impulsions selon la revendication 1 ou 2, comportant en outre une paire de comparateurs (233, 234) connectés aux bornes de l'élément à éffet Hall de manière que l'un des comparateurs produise un signal de sortie quand l'élément à effet Hall se trouve dans un champ magnétique, et qu'un courant circule dans l'élément à effet Hall.

4. Générateur d'impulsions selon la revendication 3, comprenant une porte OU (236) connectée pour recevoir les sorties desdits comparateurs et agencée pour produire un signal de sortie quand l'élément à effet Hall se trouve dans un champ magnétique et qu'un courant circule dans l'élément à effet Hall indépendamment de la polarité du champ magnétique.

5. Générateur d'impulsions selon la revendication 4, caractérisé en ce que ledit circuit bistable (265) est connecté à la sortie de ladite porte OU.

6. Générateur d'impulsions selon l'une quelconque des revendications précédentes, dans lequel ladite durée est 200 microsecondes ou moins.

7. Générateur d'impulsions selon l'une quelconque des revendications précédentes dans lequel lesdits intervalles sont supérieurs à 0,1 seconde.

8. Générateur d'impulsions selon l'une quelconque des revendications précédentes, dans lequel lesdits intervalles sont pratiquement égaux aux intervalles entre lesdites impulsions de stimulation.

9. Générateur d'impulsions selon la revendication 8, comprenant un oscillateur avec une période qui est réduite par rapport à l'intervalle entre les impulsions de stimulation, un circuit de sortie pour produire des impulsions de stimulation et un circuit d'amplificateur pour détecter des battements cardiaques naturels et comprenant un dispositif qui produit des signaux de temporisation réagissant audit circuit de sortie et audit circuit d'amplificateur en produisant un signal de temporisation d'une durée liée à la période dudit oscillateur quand un battement cardiaque naturel est détecté ou lorsqu'une impulsion de sortie de stimulation est produite.

*Fig.1*

DIGITAL CIRCUIT

HALL
XTAL
VCO
VCO ENABLE
AMP
BLANK
RECHARGE
RATE LIMIT
BATTERY

ANALOG CIRCUIT
(BATT STATUS)
(CRYSTAL CLOCK)
(VCO)
(QRS AMP)
(RATE LIMIT)
OUTPUT

*Fig.2*

| FIG.2a | FIG.2b |
| --- | --- |

## Fig.2a

Fig.2b

EP 0 030 135 B2

3

Fig. 3